# EUROPEAN PATENT APPLICATION

(11) **EP 4 729 091 A1**
(43) Date of publication of application: **22.04.2026**
(21) Application number: 24842828.6
(22) Date of filing: 29.05.2024
(51) Int. Cl.: A61M 5/152

(54) **INFUSION DEVICE**

(30) Priority: 18.07.2023 JP 2023117055
(71) Applicant: Nipro Corporation, Settsu-shi, Osaka 566-8510 (JP)
(72) Inventor: SAKAMOTO, Shingo, Settsu-shi, Osaka 566-8510 (JP)
(74) Representative: Germain Maureau
(86) International application number: PCT/JP2024/019696
(87) International publication number: WO 2025/018032

(57) **Abstract**

An infusion device 1 used for continuously feeding a medicinal solution is provided. The infusion device 1 includes a port 7 through which the medicinal solution flows in and out to and from a balloon 6 housed in a bag 5. The port 7 includes a rod portion 28 that extends from a support portion 26S that supports the balloon 6 inside the balloon 6, a connecting portion 26C in which a liquid inlet and a liquid outlet for the medicinal solution are provided, and a cover portion 25 that is fitted to the connecting portion 26C. The rod portion 28 together with the balloon 6 is housed in the body portion 15 via the neck portion 16 of the bag 5 and the port 7 is attached to the bag 5 by fitting the cover portion 25 to the connecting portion 26C in a state where the neck portion 16 is interposed therebetween.

## Description

### TECHNICAL FIELD

A technology disclosed herein relates to an infusion device suitable for administering a physiological saline solution or the like.

### BACKGROUND ART

An infusion system that, in order to administer a physiological saline solution stored in an infusion bag, the infusion bag is pressurized using a foam body that expands to enable continuous feeding of the solution is disclosed in Patent Document 1.

In the infusion system described above, the infusion bag and the foam body are arranged such a total volume thereof is maintained substantially constant throughout feeding of the solution, so that feeding of the solution at a constant flow rate is enabled. However, expansion of the foam body is easily affected by an external factor, such as temperature or the like. Accordingly, there are issues regarding reproducibility and practical implementation is considered difficult.

Regarding the technology disclosed herein, a device (infuser) that fills a reversibly elastically deformable bag member (balloon) with a medicinal solution and continuously feeds the solution using a contraction force of the balloon is known. The infuser is excellent in portability, can stably administer a small volume of the medicinal solution for a long period of time, and therefore, is widely used in medical fields, such as chemotherapy or the like.

Generally, many infusers are of the type where the balloon is housed within a mini-bottle-sized hard case. The medicinal solution is filled into the balloon through a liquid inlet of a port provided in the hard case. By doing so, the balloon is expanded and the medicinal solution is stored in the balloon in a pressurized state.

A liquid feeding tube including a connector that is connected to a winged needle or the like at an end thereof is connected to a liquid outlet of the port. A middle portion of the liquid feeding tube is equipped with a flow rate control portion, a filtration filter, a clamp, or the like. The medicinal solution stored in the balloon is fed at a constant flow rate through the liquid feeding tube by opening the clamp and adjusting the flow rate control unit.

An example of this type of infuser is disclosed in Patent Document 2. In the infuser, the case that houses the balloon is formed by recess and protrusion fitting a lid body having a liquid inlet and a liquid outlet each being bifurcated in a V-shape to a cylindrical body. A ventilation hole through which air in the case is discharged is formed in the lid body.

### CITATION LIST

### PATENT DOCUMENT

PATENT DOCUMENT 1: Japanese Unexamined Patent Publication No. 2004-73230
PATENT DOCUMENT 2: Japanese Unexamined Patent Publication No. H08-196628

### SUMMARY OF THE INVENTION

### TECHNICAL PROBLEM

Administration of a physiological saline solution is an effective treatment for a patient suffering from dehydration. This treatment is particularly effective in severe cases.

Intravenous drip is a common method for administering a physiological saline solution. However, since, in intravenous drip, the physiological saline solution is fed from an infusion bag suspended at a predetermined height using gravity, a treatment place is restricted, and the patient has difficulty in moving. Equipment, such as a suspension stand or the like, is also needed. Therefore, intravenous drip has a disadvantage of lacking portability and convenience.

In contrast, by using an infuser in administering a physiological saline solution, precise adjustment to a constant flow rate is enabled, the treatment place is not restricted, and the patient can easily move. No large-scale equipment is needed. Accordingly, it is excellent in portability and convenience to use the infuser

However, compared to administering a medicinal solution of chemotherapy or the like, in administering a physiological saline solution, administration of a large volume of the physiological saline solution is required while required precision is not high. Therefore, there is excess and shortage in use of a known infuser in administering the physiological saline solution, and room for improvement is recognized.

Therefore, this specification discloses a technology related to an infusion device suitable for administering a large volume of a medicinal solution.

### SOLUTION TO THE PROBLEM

The technology disclosed herein relates to an infusion device used for continuously feeding a medicinal solution.

The infusion device includes a reversibly elastically deformable balloon that stores the medicinal solution and includes an opening portion, a port through which the medicinal solution flows in and out to and from the balloon, and a bag that stores the balloon.

The port includes a support portion that supports the opening portion of the balloon, a rod portion that extends from the support portion inside the balloon, a connecting portion that is formed such that an area of a cross section thereof in an axis orthogonal direction that is orthogonal to a center axis of the rod portion is larger than that of the support portion and in which a liquid inlet and a liquid outlet for the medicinal solution are provided, and a cover portion that is fitted to the connecting portion from an outer side.

The bag includes a pouch-like body portion and a neck portion provided at one end of the body portion to cover the connecting portion. The rod portion together with the balloon is housed in the body portion via the neck portion and the port is attached to the bag by fitting the cover portion to the connecting portion in a state where the neck portion is interposed therebetween.

That is, according to the infusion device, similar to a known infuser, the medicinal solution is stored in the balloon, the medicinal solution can be continuously administered, and excellent portability and convenience is achieved. Since the balloon is housed in a soft pouch-like bag, not in a hard case, even when the balloon has s large volume, the balloon can be made compact during transport or disposal and is easy to handle.

Furthermore, since the port is attached to the bag by fitting the cover portion to the connecting portion in a state where the neck portion of the bag is interposed therebetween, a high degree of freedom in combination of materials of the bag and the port is achieved. Even when the materials differ, the port can be securely fixed to the bag and excellent assembly workability is also achieved.

In a fitting portion including an outer surface of the connecting portion and an inner surface of the cover portion, recesses and protrusions that clamp the neck portion may be provided on at least one of the outer surface of the connecting portion and the inner surface of the cover portion.

Thus, the port is less likely to slip out because of the recesses and protrusions, and therefore, the bag and the port can be more securely fixed.

At least one of the connecting portion and the cover portion may be formed such that the recesses and protrusions extend in a circumferential direction of the center axis.

Thus, a direction in which the recesses and protrusions extend is orthogonal to a slipping direction, and therefore, the bag and the port can be even more securely fixed.

The bag may be formed by joining edge portions of a pair of synthetic resin sheets that are superimposed on each other at a predetermined width, except for respective portions of the pair of synthetic resin sheets that correspond to an opening of the neck portion.

Thus, a bulk of the bag in a state where the bag is not expanded can be reduced.

The neck portion may be three-dimensionally formed such that an open state is maintained.

Thus, in assembling the port to the bag, it is assumed that the port and the bag are inserted through the opening of the neck portion, and with the opening opened then, the port and the balloon can be easily inserted. Accordingly, excellent assembly workability is achieved.

The connecting portion may include a flange portion that protrudes in the axis orthogonal direction and extends in the circumferential direction of the center axis, and the flange portion may receive an opening end of the neck portion and be fitted to the cover portion.

Thus, stable positioning of the port relative to the bag and the cover portion in the axial direction can be performed. Accordingly, excellent assembly workability is achieved.

A slope portion inclined downward toward the support portion may be provided in a portion of the connecting portion located closer to the support portion.

Thus, smooth guidance via the slope portion can be provided, and therefore, the rod portion and the balloon can be easily inserted through the opening of the neck portion. Accordingly, excellent assembly workability is achieved.

A pair of outer longitudinal grooves that extend in a direction of the center axis may be formed so as to face each other on the inner surface of the cover portion that is fitted to the outer surface of the connecting portion, and a pair of joint allowances that protrude from both sides of the neck portion may be housed in the outer longitudinal grooves when the cover portion is fitted to the connecting portion.

In putting the neck portion between the connecting portion and the cover portion to be clamped, the joining flange may be an obstruction, but the joint allowances can be housed in the outer longitudinal grooves and thus do not become an obstacle. Accordingly, biting and damage of the neck portion can be suppressed and excellent assembly workability is achieved.

A positioning structure that fixes relative positions of the connecting portion and the cover portion in the circumferential direction of the center axis may be provided between the connecting portion and the cover portion.

Thus, the cover portion is not displaced in the circumferential direction relative to the connecting portion, and therefore, the cover portion can be properly positioned simply by fitting the cover portion to the connecting portion.

An inner longitudinal groove that extends in a direction of the center axis may be formed on an outer surface of the connecting portion, and the inner longitudinal groove may constitute a ventilation passage via which an outer region of the balloon inside the bag communicates with an outside environment.

Thus, even when air is present inside the bag, the air can be discharged. Accordingly, the balloon can be freely expanded until the balloon is regulated by the bag and can be filled with a predetermined volume of the medicinal solution.

A hole through which a strap can be inserted may be formed in the cover portion.

Thus, the infusion device can be hung and locked around or over the neck or the shoulder of a patient. Accordingly, portability of the infusion device during use can be increased.

The infusion device may further include a tube assembly that transfers the medicinal solution that is pushed out from the balloon, and the tube assembly may include a liquid feeding tube one end of which is connected to the liquid outlet and that includes a connector at the other end, and a flow rate setting portion that sets a flow rate of the medicinal solution that is fed, and the flow rate setting portion may be formed of an orifice tube provided in a portion of the liquid feeding tube and having specific length and flow passage cross section.

When the tube assembly is the orifice tube, a relatively high flow rate can be used to stably set the flow rate. This requires only using a tube having a specific dimension in accordance with the required flow rate, a flow rate control unit with a complex structure as in a known infuser is unnecessary. Accordingly, member cost and the number of parts can be reduced.

The bag may be formed of a soft material.

Thus, excellent portability can be achieved.

### ADVANTAGES OF THE INVENTION

According to the technology disclosed herein, a larger volume of a medicinal solution can be continuously administered than a volume of a medicinal solution that can be fed by a known infuser. Therefore, an infusion device to which the technology disclosed herein is applied is suitable for administering a physiological saline solution or the like and is effective in treatment of dehydration or the like.

### BRIEF DESCRIPTION OF THE DRAWINGS

[FIG. 1] FIG. 1 is a schematic perspective view of an infusion device to which the technology disclosed herein is applied.
[FIG. 2] FIG. 2 is a view used for describing a main portion of a bag.
[FIG. 3] FIG. 3 is a schematic cross-sectional view of a portion including a balloon and a port taken along an arrow A1-A1 of FIG. 1.
[FIG. 4] FIG. 4 is an enlarged view of a main portion of FIG. 3.
[FIG. 5] FIG. 5 is an exploded perspective view of the port.
[FIG. 6] FIG. 6 is a view used for describing structures of a cover portion and the port.
[FIG. 7] FIG. 7 is a schematic cross-sectional view of a fitting portion of the port and the cover portion.
[FIG. 8] FIG. 8 is a view used for describing use of the infusion device.
[FIG. 9] FIG. 9 is a view used for describing a modified example of the infusion device.

### DESCRIPTION OF EMBODIMENTS

A technology disclosed herein will be described. However, the following description provides a mere example by nature.

### <Structure Of Infusion Device>

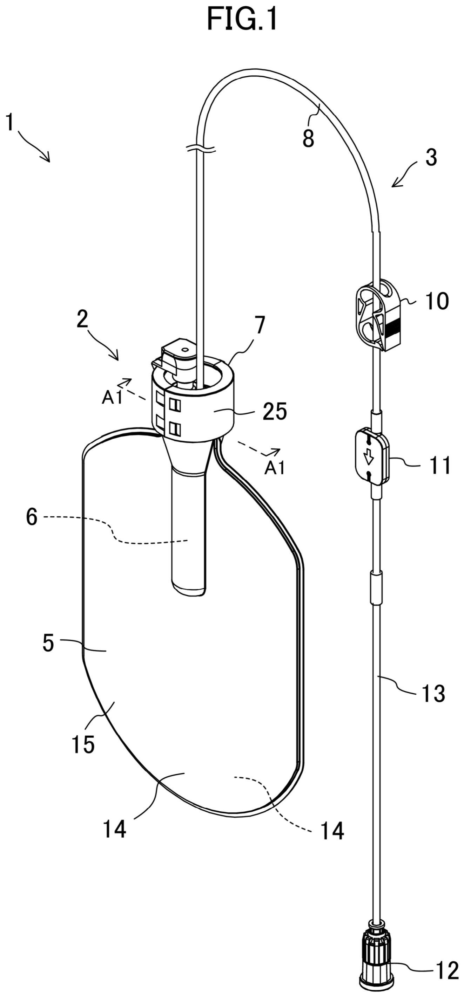

FIG. 1 illustrates an overall structure of an infusion device 1 to which the technology disclosed herein is applied. The infusion device 1 is configured to stably feed a medicinal solution continuously, that is, over a long period of time, in order to perform infusion (subcutaneous, intravenous, or intraperitoneal administration of liquid, or the like). Specifically, the infusion device 1 is devised to allow, while using advantages of known infusers, such as portability and convenience, administration of a large volume of a medicinal solution at relatively high-speed, reduction in the number of parts, easy assembly, and providing the device at a low cost.

An example of a medicinal solution suitable for the infusion device 1 is, as described above, a physiological saline solution. However, the medicinal solution is not limited to the physiological saline solution, and any medicinal solution requiring large-volume administration can be a target for use of the infusion device 1.

The infusion device 1 substantially includes a bag assembly 2 and a tube assembly 3. The bag assembly 2 includes components, such as a bag 5, a balloon 6, a port 7, or the like. The tube assembly 3 includes components, such as a liquid feeding tube 8, a clamp 10, a filter 11, or the like.

As illustrated in FIG. 1, the infusion device 1 is commercialized in a state where the bag assembly 2 and tube assembly 3 are integrally assembled. Depending on specifications, the bag assembly 2 and tube assembly 3 may be manufactured separately to be commercialized.

The bag assembly 2 is configured to store a medicinal solution and push out the medicinal solution using a pressure accompanying contraction of the balloon 6 as will be described later. The tube assembly 3 is configured to transfer the medicinal solution pushed out from the balloon 6.

### (Tube Assembly)

The liquid feeding tube 8 is formed of a tube made of silicon or the like. An upstream end of the liquid feeding tube 8 is connected to a liquid outlet of the port 7. A connector 12 having a locking function and an air vent function is attached to a downstream end of the liquid feeding tube 8. During use of the infusion device 1, the connector 12 is connected to a winged needle or the like.

The clamp 10 is mounted on midway of the liquid feeding tube 8. The clamp 10 opens and closes a flow passage of the liquid feeding tube 8 by a manual operation. The filter 11 is arranged on midway of the passage of the liquid feeding tube 8. Even when foreign matters or bacteria are mixed into the medicinal solution, the foreign matters or the bacteria are removed as the medicinal solution passes through the filter 11.

A flow rate setting portion that sets a flow rate of the medicinal solution that is fed is provided at a downstream side of the liquid feeding tube 8. The flow rate setting portion is formed of an orifice tube 13 having specific length and flow passage cross section. That is, the length and the flow passage cross section are specified through experiments or the like in advance in response to change in pressure of the medicinal solution accompanying contraction of the balloon 6 in order to ensure flow of the medicinal solution at a predetermined flow rate while satisfying required precision.

Thus, the orifice tube 13 is set to a specific length and passage cross sectional in accordance with a required flow rate. It is only required to use a tube having specified dimensions, and a complex flow rate control portion as in a known infuser is not needed. Therefore, member cost and the number of parts can be reduced.

### (Bag)

The bag 5 is a soft case that houses the balloon 6. The bag 5 is formed by joining a pair of flexible synthetic resin sheets 14 formed of a material, such as soft polyvinyl chloride (PVC) or the like, through heat welding, ultrasonic welding, or the like. The bag is soft, and therefore, can be compactly deformed in a manner in which a body portion 15 that will be described later is rolled or folded when the medicinal solution is not filled therein, when the bag is carried after administration of the physiological saline solution of the infusion device 1, or the like and is excellent in portability, as compared to a hard bag. In particular, when the infusion device 1 is used with the physiological saline solution, a volume of the physiological saline solution is larger than a volume of the physiological saline solution used with a known infuser, and therefore, a volume of the infusion device 1 itself is increased. In this case, when the bag is soft, the bag is excellent in portability as described above and therefore is more preferable.

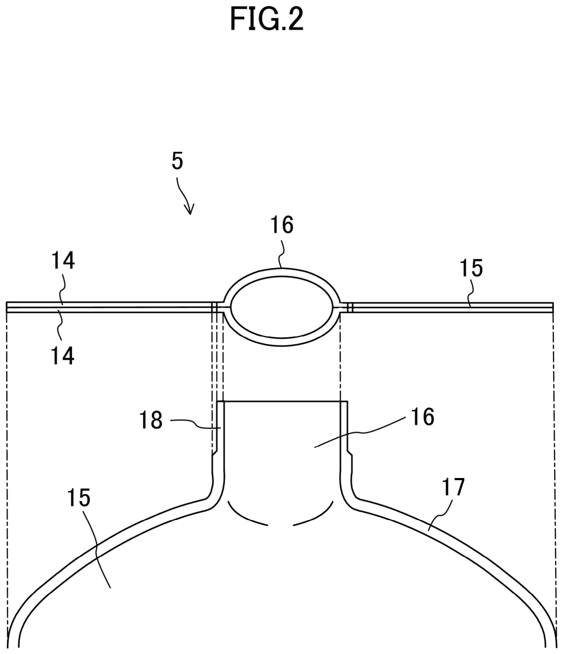

Specifically, as illustrated in FIG. 1 and FIG. 2, the bag 5 includes the pouch-like body portion 15 having a substantially oval shape and a neck portion 16 provided so as to protrude from one end of the body portion 15. The bag 5 is manufactured, for example, in the following manner.

The pair of synthetic resin sheets 14 are superimposed on each other and then are cut so that the body portion 15 and the neck portion 16 are formed. Respective edge portions of the cut pair of synthetic resin sheets 14 are joined at a predetermined width, except for respective portions of the pair of synthetic resin sheets 14 that correspond to an opening of the neck portion 16. Thus, a joint allowance 17 extending along an edge of the bag 5 is formed at an outer peripheral portion of the bag 5.

Therefore, the bag 5 before assembling the port 7 and the balloon 6 is in a state where the pair of soft synthetic resin sheets 14 are superimposed on each other in both the body portion 15 and the neck portion 16. Accordingly, the bag 5 has an advantage that the bag 5 is formed to be flat except for a portion where the port 7 and the balloon 6 are assembled and thus the bag 5 is not bulky during transport of the infusion device 1 or when the bag is discarded after use.

More preferably, as illustrated in FIG. 2, the neck portion 16 of the infusion device 1 may be three-dimensionally formed by vacuum forming or the like such that an open state of the opening is kept. Thus, the rod portion 28 of the port 7 and the balloon 6 can be easily inserted into the neck portion 16. Accordingly, it is not needed to expand and open the neck portion 16 from a state where the soft synthetic resin sheets 14 are superimposed on each other in order to insert the port 7, and therefore, excellent assembly workability is achieved.

Each of the pair of joint allowances 17 that project on both sides of the neck portion 16 is provided with a narrow width portion 18 formed by cutting away a portion thereof or the like. Specifically, each of narrow width portions 18 is provided within a predetermined range extending from an opening end of the neck portion 16 toward a base end (the range that covers an outer surface of a connecting portion 26C that will be described later).

### (Balloon, Port)

The balloon 6 is a member that stores the medicinal solution. The port 7 is a member that causes the medicinal solution to flow into and out of the balloon 6.

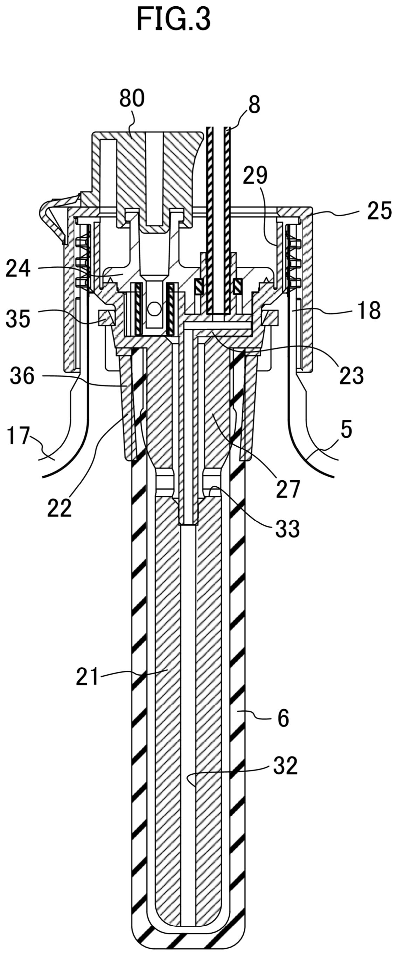

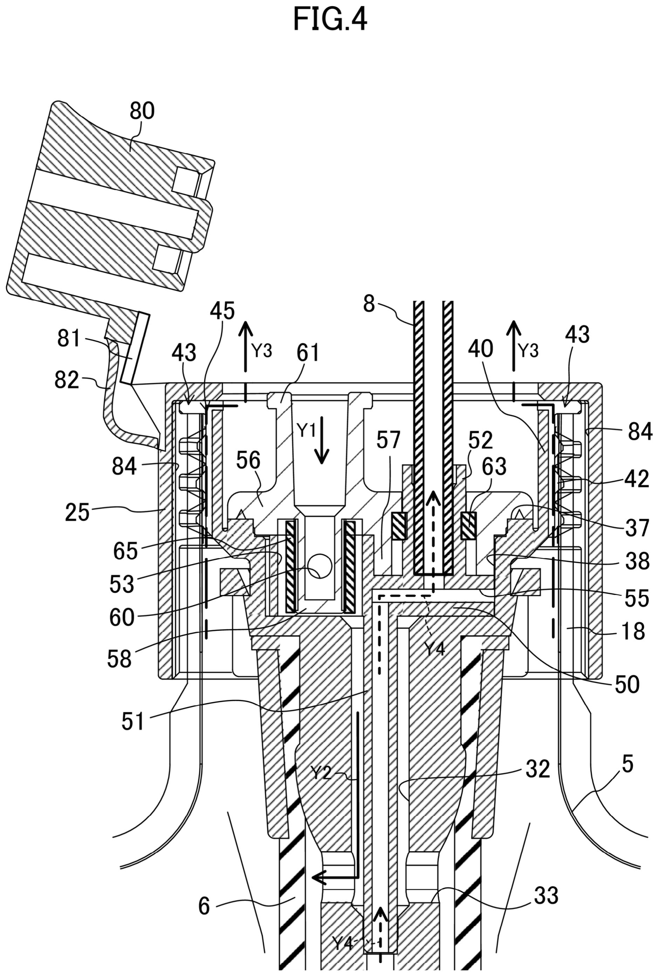

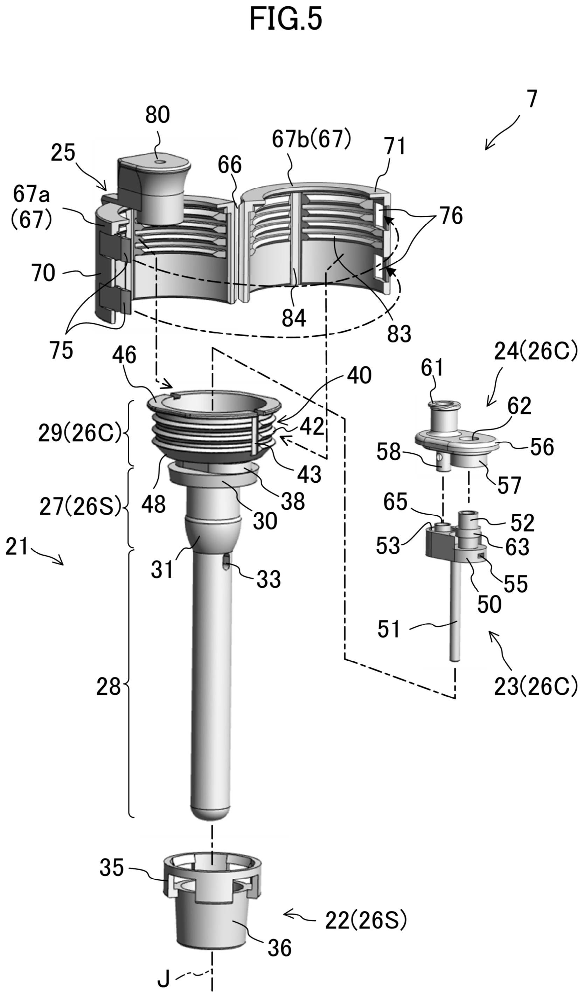

FIG. 3 illustrates a schematic cross-sectional view of a portion including the balloon 6 and the port 7 taken along the arrow A1-A1 in FIG. 1. FIG. 4 illustrates an enlarged view of a main portion of FIG. 3 for describing a function of each portion. FIG. 5 illustrates an exploded perspective view of the port 7.

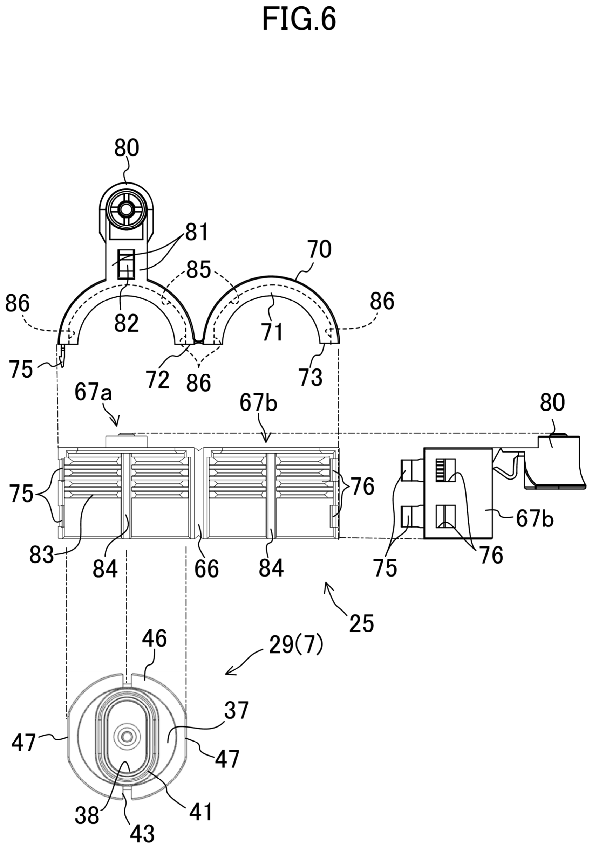

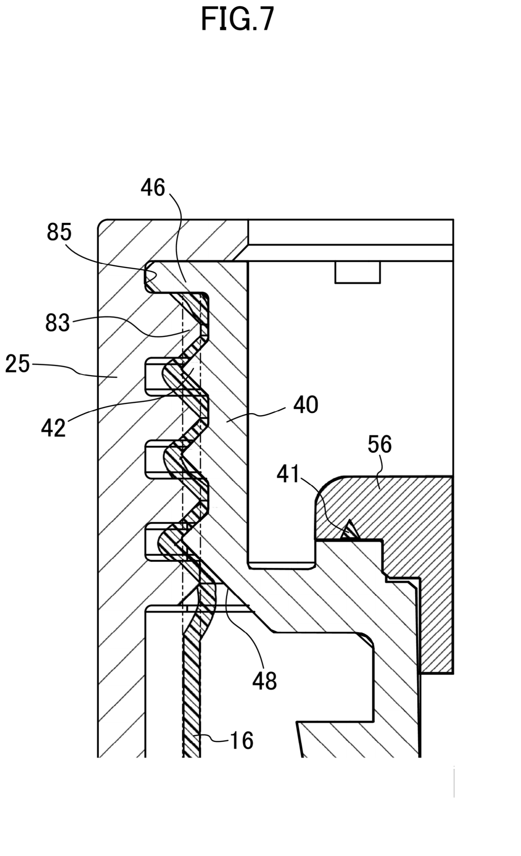

FIG. 6 illustrates a cover portion 25 and the port 7. A view at center is a front view of the cover portion 25 before assembly, a view at top is a top view of the cover portion 25, and a view at right is a right side view of the cover portion 25. A view below the front view of the cover portion 25 is a top view of the port 7 (a third member 23 and a fourth member 24 that will be described later are omitted). FIG. 7 illustrates a schematic cross-sectional view of a fitting portion of the port 7 and the cover portion 25.

As illustrated in FIG. 5, the port 7 includes a first member 21, a second member 22, the third member 23, the fourth member 24, the cover portion 25, and the like. The first member 21, the second member 22, the third member 23, the fourth member 24, and the cover portion 25 are molded products formed of a hard synthetic resin, such as polypropylene (PP) or the like.

The first member 21 is a member that constitutes a main body of the port 7 and includes a short cylindrical inner support portion 27, a long cylindrical rod portion 28 having a smaller diameter than that of the inner support portion 27 and extending from one end of the inner support portion 27, and a short cylindrical fitting portion 29 having a larger diameter than that of the inner support portion 27 and provided at the other end of the inner support portion 27. Center axes J of the inner support portion 27, the rod portion 28, and the fitting portion 29 coincide.

The fitting portion 29 together with the third member 23 and the fourth member 24 constitutes a connecting portion. The inner support portion 27 together with the second member 22 constitutes a support portion. For convenience, in the following description, the fitting portion 29 or the like will be also referred to as the "connecting portion 26C," and the inner support portion 27 or the like will be also referred to as a "supporting portion 26S." Moreover, a direction of the center axis J is referred to as an "axial direction," a direction orthogonal to the center axis J is referred to as an "axis orthogonal direction," and directions around the center axis J are referred to as a "circumferential direction," a "radial direction," or the like.

An annular flange portion 30 protruding in the axis orthogonal direction is formed on a portion of the inner support portion 27 located closer to the fitting portion 29. An expanded portion 31 that has been slightly expanded outward in a radial direction is formed on a portion of the inner support portion 27 located closer to the rod portion 28.

As illustrated in FIG. 3, a longitudinal liquid passage 32 is formed in the inner support portion 27 and the rod portion 28 so as to extend therethrough in the axial direction at center thereof. The longitudinal liquid passage 32 in the rod portion 28 is formed so as to have a smaller diameter than that of the longitudinal liquid passage 32 in the inner support portion 27. A lateral liquid passage 33 is formed at a boundary between the inner support portion 27 and the rod portion 28 so as to pass therethrough in the axis-orthogonal direction and communicate with the longitudinal liquid passage 32.

As illustrated in FIG. 3 and FIG. 5, the second member 22 is a member that is assembled to the first member 21 so as to cover the inner support portion 27. The second member 22 includes a press-fit frame portion 35 that is fixed by being press-fitted into the flange portion 30 and a cylindrical outer support portion 36 having an inner diameter slightly larger than an outer diameter of the inner support portion 27.

The balloon 6 is formed of a synthetic resin material, such as rubber or the like, so as to be reversibly elastically deformable. As illustrated in FIG. 1 and FIG. 3, the balloon 6 in an unloaded state has a tube-like shape and is put over the rod portion 28. The opening portion of the balloon 6 is press-fitted into the inner support portion 27. The second member 22 is further put thereover.

Then, the press-fit frame portion 35 is press-fitted into the flange portion 30 and the second member 22 is assembled to the first member 21, so that the opening portion of the balloon 6 is crushed in a state of being sandwiched between the outer support portion 36 and the inner support portion 27. Thus, the balloon 6 is supported by the inner support member 27 and the outer support member 36 (that is, a support portion 26S) in a state where the opening portion thereof is blocked.

As a result, of the port 7, the rod portion 28 is housed so as to extend from the support portion 26S inside the balloon 6 and the connecting portion 26C faces an outer side of the balloon 6 from the support portion 26S. The rod portion 28 covered by the balloon 6 is housed in the body portion 15 via the neck portion 16. Furthermore, a portion of the neck portion 16 at a tip end side covers an outer surface of the connecting portion 26C, so that the cover portion 25 is fitted to the connecting portion 26C with the neck portion 16 interposed therebetween. Thus, the port 7 is attached to the bag 5.

Specifically, as illustrated in FIG. 4, FIG. 5, and FIG. 6, the fitting portion 29 includes a circular bottom wall portion 37, a short cylindrical housing tube portion 38 connected to the bottom wall portion 37 in an open state, and a short cylindrical cylinder wall portion 40. A cross-sectional shape of the housing tube portion 38 is elliptical. A seal ring 41 is attached around an opening of the housing tube portion 38 in the bottom wall portion 37. The fitting portion 29 is connected to the inner support portion 27 via the housing tube portion 38.

On an outer surface of the cylindrical wall portion 40, multiple (three in this embodiment) inner projecting ridge portions 42 that protrude outward in the radial direction and extend in the circumferential direction are formed so as to align in the axial direction. As illustrated in FIG. 7, a maximum outer diameter of the cylindrical wall portion 40 is slightly larger than an inner diameter of the neck portion 16, and a minimum outer diameter of the cylindrical wall portion 40 is slightly smaller than the inner diameter of the neck portion 16. Thus, when the cylindrical wall portion 40 is inserted (press-fitted) in the neck portion 16, the neck portion 16 is closely fitted to the cylindrical wall portion 40 and engages into recessed portions each being provided between every adjacent ones of the inner projecting ridge portions 42.

A pair of inner longitudinal grooves 43 extending in the axial direction are also formed on the outer surface of the cylindrical wall portion 40 in a line-symmetrical manner with respect to the center axis J. Each of the inner longitudinal grooves 43 is formed so as to pass through the inner projecting ridge portions 42. Accordingly, even when the neck portion 16 is closely fitted to the cylindrical wall portion 40, as illustrated in FIG. 4, a gap 45 (a ventilation passage that will be described later) extending in the axial direction is ensured inside the neck portion 16 because of presence of the inner projecting ridge portions 42.

An annular flange portion 46 is provided at a tip end portion of the cylindrical wall portion 40 so as to protrude from an edge of the tip end portion in the axis orthogonal direction and extend in the circumferential direction. As illustrated in FIG. 7, an outer diameter of the flange portion 46 is larger than the inner diameter of the neck portion 16. Accordingly, in inserting the port 7 into the bag 5, the opening end of the neck portion 16 is received by the flange portion 46. Thus, the port 7 can be positioned in a proper position in the axial direction relative to the bag 5.

The inner longitudinal grooves 43 are formed to pass through also the flange portion 46. Accordingly, the gap 45 that is ensured by the inner longitudinal grooves 43 also passes through the flange portion 46.

As illustrated in FIG. 6, a pair of notch portions 47 (constituting a positioning structure) each of which is formed by cutting out a portion of the flange portion 46 along a chord in a direction orthogonal to the inner longitudinal grooves 43 as viewed from the axial direction are provided in the flange portion 46.

An area of a cross section of the connecting portion 26C in the axis orthogonal direction is larger than that of the support portion 26S (that is, the inner support portion 27 with the second member 22 assembled thereto) in the axial direction. Therefore, an inclined portion 48 (slope portion) is formed so as to be inclined downward toward the support portion 26S in a portion of the connecting portion 26C located closer to the support portion 26S, as illustrated in FIG. 5 and FIG. 7, in order to make it easy to insert the neck portion 16.

The third member 23 is a member that is assembled to the first member 21 from a side where the fitting portion 29 is present. As illustrated in FIG. 4 and FIG. 5, the third member 23 includes a base portion 50 that is fitted into the housing tube portion 38 and a narrow and long thin tube portion 51 extending from a bottom surface of the base portion 50 in the axial direction. The thin tube portion 51 is inserted in the longitudinal liquid passage 32 by assembling the third member 23 to the first member 21. A tip end of the thin tube portion 51 is press-fitted into the vertical fluid passage 32 at a position closer to a tip end of the rod portion 28 than the lateral liquid passage 33.

An insertion tube 52 (constituting a liquid outlet for the medicinal solution) is provided at one end of a top surface of the base portion 50. A cylindrical valve hole 53 is provided at the other end of the top surface of the base portion 50. A lateral hole 55 communicating with the thin tube portion 51 and the insertion tube 52 is formed inside the base portion 50.

The fourth member 24 is a member that is assembled on the base portion 50 and includes a lid portion 56 that is fitted into the bottom wall portion 37 so as to seal the housing tube portion 38. An annular frame portion 57 that receives the insertion tube 52 and a valve cylinder body 58 having a bottomed cylindrical shape that is inserted into the valve hole 53 are provided on a bottom surface of the lid portion 56. A liquid outlet hole 60 is formed in the valve cylinder body 58 so as to pass through a side surface of the valve cylinder body 58.

A cylindrical inlet port 61 (constituting a liquid inlet for the medicinal solution) that communicates with inside of the valve cylinder 58 and an insertion hole 62 into which the insertion tube 52 is inserted are formed on a top surface of the lid portion 56. A space between the annular frame portion 57 and the insertion hole 6 is sealed by an O-ring 63 that is mounted on the insertion tube 52. The valve cylinder body 58 is housed in the valve hole 53 in a state where a silicone tube 65 that constitutes a check valve is mounted thereon.

The liquid inlet and the liquid outlet for the medicinal solution are provided in the connecting portion 26C by assembling the third member 23 and the fourth member 24 to the first member 21,.

### (Cover Portion)

The cover portion 25 is a member that is fitted to the connect part 26C from an outer side, as illustrated in FIG. 5 and FIG. 6. The cover portion 25 of this embodiment includes a pair of split parts 67 (a first split part 67a and a second split part 67b) connected by a hinge 66. Each of the split parts 67 is fitted to the connector portion 26C in the axis orthogonal direction.

Each of the split parts 67 includes a semicylindrical wall portion 70 having a semicylindrical shape and a semicircular edge portion 71 protruding inward from an upper end of the semicylindrical wall portion 70 in the radial direction. Respective one side portions (connecting side portions 72) of the semicylindrical wall portions 70 are connected to the other via the hinge 66.

A pair of engagement claws 75 are provided on the other side portion (an engagement side portion 73) of the semi-cylindrical wall portion 70 of the first split part 67a. A pair of engagement grooves 76 are provided on an engagement side portion 73 of the semi-cylindrical wall portion 70 of the second split part 67b. The two split parts 67 are connected by bending the hinge 66, bringing the engagement side portions 73 of the semi-cylindrical wall portions 70 of the respective split parts 67 into contact, and inserting the engagement claws 75 into the engagement grooves 76.

Thus, the cylindrical cover portion 25 is formed. The outer peripheral surface of the cover portion 25 is used as a sticking position of an information label on which required information for the infusion device 1, such as a product name, an item number, a lot, or the like, is written.

As illustrated in FIG. 4 and FIG. 6, a cap 80 that covers the inlet port 61 so as to freely open and close the inlet port 61 is attached to the first split part 67a. The cap 80 is integrally molded to the first split part 67a via a pair of support arms 81 and a spring hinge 82. The cap 80 is arranged in a direction orthogonal to the connecting side portions 72 and the engagement side portion73 as viewed in the axial direction.

Multiple (four in this embodiment) outer projecting ridge portions 83 that are fitted to the inner side ridge portions are formed on an inner side surface of each of the semicylindrical wall portions 70. Specifically, each of the outer projecting ridge portions 83 protrudes inward in the radial direction, extends in the circumferential direction, and is arranged such that the outer projecting ridge portion 83 and the inner projecting ridge portion 42 are alternately arranged in the axial direction (the inner projecting ridge portions 42 and the outer projecting ridge portions 83 constitute recesses and protrusions).

An outer longitudinal groove 84 that extends from a lower side of the semicircular edge portion 71 in the axial direction and passes through the semicylindrical wall portion is formed on the inner surface of each of the semicylindrical wall portions 70 in a line-symmetrical manner with respect to the center axis J. Each of the outer longitudinal grooves 84 are formed by making the inner surface of each of the semicylindrical wall portions 70 including the outer projecting ridge portions 83 recessed. The outer longitudinal grooves 84 are arranged in a direction orthogonal to the connecting side portions 72 and the engagement side portion 73 as viewed in the axial direction.

As illustrated in FIG. 6 and FIG. 7, a fitting groove 85 to which the flange portion 46 is fitted is formed at an inner corner portion that is a boundary between the semicylindrical wall portion 70 and the semi-annular rim portion 71. A pair of straight portions 86 (that constitute a positioning structure) that are fitted to the notch portions 47 are formed in a portion of the fitting groove 85 where the connecting side portion 72 and the engagement side portion 73 are located.

Accordingly, a relative position of the cover portion 25 with respect to the connecting portion 26C in the circumferential direction is fixed and the each of the inner longitudinal grooves 43 and a corresponding one of the outer longitudinal groove 84 inevitably face each other. Moreover, the cover portion 25 is not displaced in the circumferential direction relative to the connecting portion 26C. The position of the cover portion 25 relative to the port 7 in the axial direction is also fixed. With the cover portion 25 fitted to the connecting portion 26C, the cap 80 can be properly positioned relative to the inlet port 61.

As described above, the pair of joint allowances 17 protrude from both sides of the neck portion 16. In fitting the cover portion 25 to the connecting portion 26C, the neck portion 16 is sandwiched between the cover portion 25 and the connecting portion 26C, and therefore, the joint allowances 17 are obstacles. In contrast, in the infusion device 1, a pair of the outer longitudinal grooves 84 are formed in the cover portion 25. Accordingly, as illustrated in FIG. 4, the pair of joint allowances 17 can be housed in the outer longitudinal grooves 84. Thus, biting of the joining ridges 17, damage of the neck portion 16, or the like can be suppressed.

Furthermore, the narrow width portion 18 is provided on the joint allowance 17 of the neck portion 16. Accordingly, biting of the joint allowance 17, damage of the neck portion 16, or the like can be further suppressed. Furthermore, the neck portion 16 can be properly clamped by the recesses and the protrusions formed of the outer projecting ridge portions 83 and the inner projecting ridge portions 42 by housing the joint allowances 17 in the outer longitudinal groove 84, as illustrated in FIG. 7. Accordingly, even when materials of the bag 5 and the port 7 differ, the port 7 can be securely fixed to the bag 5.

### <Use of Infusion Device>

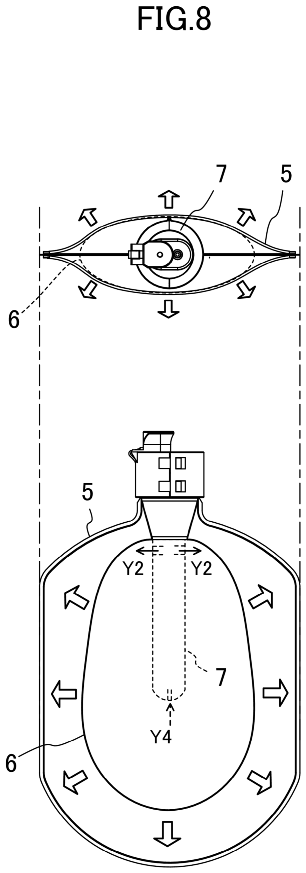

With reference to FIG. 4, FIG. 8, or the like, use of the infusion device 1 will be described. Use of the infusion device 1 is roughly classified into filling of a medicinal solution and administering the medicinal solution. Note that, in the infusion device 1 when being unused, as illustrated in FIG. 1, the bag 5 has a sheet-like shape and is lightweight and compact. Moreover, the flow passage of the liquid feeding tube 8 is closed by the clamp 10.

### (Filling With Medicinal Solution)

As indicated by the arrow Y1 in FIG. 4, the cap 80 is removed and the medicinal solution is injected through the inlet port 61 using a syringe or the like. The injected medicinal solution flows in the valve cylinder body 58 and flows out through the liquid outlet port 60 to the valve hole 53 against the silicon tube 65. Once injection of the medicinal solution stops, the liquid outlet hole 60 is closed by the silicone tube 65. Accordingly, backflow of the medicinal solution is blocked.

The valve hole 53 communicates with the longitudinal liquid passage 32 and, as indicated by the arrow Y2 in FIG. 4 and FIG. 8, the medicinal solution flows into the inside of the balloon 6 from the rod portion 28 through the outer portion of the thin tube portion 51 of the longitudinal liquid passage 32 and the lateral liquid passage 33. Thus, the balloon 6 is filled with the medicinal solution. As a volume of the filled medicinal solution increases, the balloon 6 expands, as illustrated in FIG. 8. The bag 5 also expands as the balloon 6 expands.

A ventilation passage (the gap 45 extending inside the neck portion 16 in the axial direction) is ensured by the inner longitudinal grooves 43 also in the fitting portion between the cover portion 25 and the connecting portion 26C. Furthermore, since each of the inner longitudinal grooves 43 is configured to face a corresponding one of the outer longitudinal grooves 84, the ventilation passage is stably ensured.

Thus, an outer region of the balloon 6 inside the bag 5 communicates with an outside environment via the ventilation passage. Accordingly, even when air is present inside the bag 5, the air is discharged as indicated by the arrow Y3 in FIG. 4.

Thus, the balloon 6 can expand until expansion thereof is restricted by the bag 5 and can be filled with the medicinal solution of a volume corresponding to a predetermined volume (for example, 500 ml) of the bag 5. Excessive expansion of the balloon 6 can be prevented. Although the balloon 6 becomes thinner due to expansion, damage of the balloon 6 can be suppressed since the balloon 6 is covered by the bag 5.

### (Administration Of Medicinal Solution)

The clamp 10 is operated to open a flow passage of the liquid feeding tube 8. Thus, the medicinal solution flows in the longitudinal liquid passage 32 from the tip end of the rod portion 28 with the pressure accompanying contraction of the balloon 6 as indicated by the arrow Y4 in FIG. 4 and FIG. 8. The medicinal solution that has flowed in the longitudinal liquid passage 32 flows in the thin tube portion 51 and then the liquid feeding tube 8 through the lateral hole 55 and the insertion tube 52.

A flow of the medicinal solution that has flowed in the liquid feeding tube 8 is regulated in the orifice tube 13 and the medicinal solution flows out from the connector 12 at a predetermined flow rate. Thereafter, air may be released therefrom and the connector 12 of the liquid feeding tube 8 may be connected to a winged needle or the like. The orifice tube 13 is set so that a required flow rate can be maintained until the balloon 6 shrinks and the medicinal solution is almost depleted. Accordingly, the medicinal solution can be used properly with almost no waste.

Since the infusion device 1 can be carried similar to a known infuser, treatment can be performed anywhere and the patient can easily move. Accordingly, the infusion device 1 during use offers excellent portability and convenience. Furthermore, the infusion device 1 without the medicinal solution therein is lightweight and compact. Accordingly, even when there are a large number of infusion devices 1, the infusion devices 1 do not take up much space and can be easily transported. Accordingly, the infusion device 1 when unused and during disposal also offers excellent convenience.

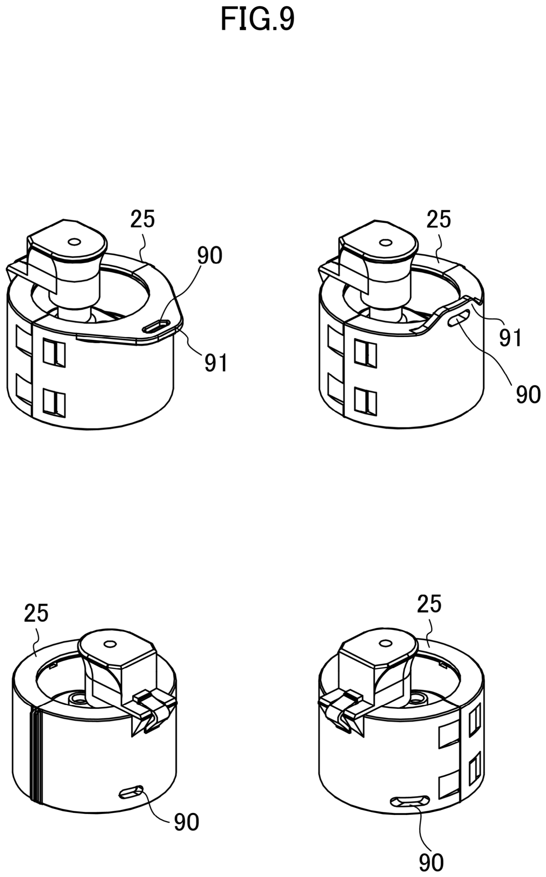

In order to increase the portability of the infusion device 1 during use, a hole, specifically, a through hole 90, through which a strap can be inserted may be formed in the cover portion 25. FIG. 9 illustrates a specific example of the through hole 90. As illustrated in a view at upper right and a view at upper left in FIG. 9, a protruding piece 91 may be provided at an end portion of the cover portion 25 and the through hole 90 may be formed therein. Furthermore, as illustrated in a view at lower right and a view at lower left in FIG. 9, the through hole 90 may be formed in a side portion of the cover portion 25. As another option, the hole may not be a through hole. For example, a hole may be provided to allow insertion and fixation of a socket-shaped end portion with a strap fixed, or a rod-shaped fixing portion may be provided near an opening of a recess-shaped hole that is opened only on one side to allow the strap to wrap around the fixing portion.

With the strap attached to the infusion device 1, the infusion device 1 can be hung and locked around or over the neck or the shoulder of the patient. Accordingly, the portability of the infusion device 1 during use can be increased.

Note that the technology disclosed herein is not limited to the above-described embodiment but also encompasses various other configurations.

As another embodiment, a fitting portion including helical recesses and protrusions may be provided between the cover portion 25 and the connecting portion 26C and configured such that the cover portion 25 is screwed in the connecting portion 26C in the axial direction. The ventilation passage may be formed also by forming a hole passing through in the axial direction in a bottom wall portion 37.

In the above-described embodiment, each of the outer peripheral surface of the connecting portion and the inner peripheral surface of the cover portion has recesses and protrusions in the circumferential direction, but recesses and protrusions in the circumferential direction may be provided in one of the outer peripheral surface of the connecting portion and the inner peripheral surface of the cover portion. In this case, for example, by forming either one of the outer peripheral surface of the connecting portion or the inner peripheral surface of the cover portion into a flat structure without recesses and protrusions, a degree of molding freedom of the one of the outer peripheral surface of the connecting portion or the inner peripheral surface of the cover portion is increased, and therefore, the infusion device 1 can be efficiently formed.

In the above-described embodiment, each of the inner longitudinal grooves 43 and a corresponding one of the outer longitudinal grooves 84 face each other, but each of the inner longitudinal grooves 43 and a corresponding one of the outer longitudinal grooves 84 may not necessarily face each other. However, it is more preferable that each of the inner longitudinal grooves 43 and a corresponding one of the outer longitudinal grooves 84 face each other, because this allows the joint allowance 17 to be aligned with portions of the inner longitudinal grooves 43 when inserting the neck portion 16 of the bag 5 into the port 7, so that aligning of the joint allowance 17 can be easily aligned with the outer longitudinal grooves 84 of the cover 25 and a space of the ventilation passage can be ensured without recesses and protrusions provided in each groove portion.

### INDUSTRIAL APPLICABILITY

### DESCRIPTION OF REFERENCE CHARACTERS

- 1: Infusion device
- 2: Bag assembly
- 3: Tube assembly
- 5: Bag
- 6: Balloon
- 7: Port
- 8: Liquid feeding tube
- 10: Clamp
- 11: Filter
- 12: Connector
- 13: Orifice tube (flow rate setting portion)
- 15: Body portion
- 16: Neck portion
- 17: Joint allowance
- 18: Narrow width portion
- 21: First member
- 22: Second member (support portion)
- 23: Third member (connecting portion)
- 24: Fourth member (connecting portion)
- 25: Cover portion
- 26C: Connecting portion
- 26S: Support portion
- 27: Inner support portion (support portion)
- 28: Rod portion
- 29: Fitting portion
- 32: Longitudinal liquid passage
- 33: Lateral liquid passage
- 36: Outer support portion
- 40: Cylinder wall portion
- 42: Inner projecting ridge portion (recesses and protrusions)
- 43: Inner longitudinal groove
- 45: Gap (ventilation passage)
- 46: Flange portion
- 47: Notch portion (positioning structure)
- 48: Inclined portion (slope portion)
- 52: Insertion tube (liquid outlet for medicinal solution)
- 60: Liquid outlet hole
- 61: Inlet port (liquid inlet for medicinal solution)
- 66: Hinge
- 67: Split part
- 80: Cap
- 83: Outer projecting ridge portion (recesses and protrusions)
- 84: Outer longitudinal groove
- 86: Stright portion (positioning structure)
- J: Center axis

## Claims

1. An infusion device used for continuously feeding a medicinal solution, the infusion device comprising:
a reversibly elastically deformable balloon that stores the medicinal solution and includes an opening portion;
a port through which the medicinal solution flows in and out to and from the balloon; and
a bag that stores the balloon,
wherein
the port includes
a support portion that supports the opening portion of the balloon,
a rod portion that extends from the support portion inside the balloon,
a connecting portion that is formed such that an area of a cross section thereof in an axis orthogonal direction that is orthogonal to a center axis of the rod portion is larger than that of the support portion and in which a liquid inlet and a liquid outlet for the medicinal solution are provided, and
a cover portion that is fitted to the connecting portion from an outer side, the bag includes
a pouch-like body portion,
a neck portion provided at one end of the body portion to cover the connecting portion, and
the rod portion together with the balloon is housed in the body portion via the neck portion and the port is attached to the bag by fitting the cover portion to the connecting portion in a state where the neck portion is interposed therebetween.

2. The infusion device of claim 1, wherein
in a fitting portion including an outer surface of the connecting portion and an inner surface of the cover portion, recesses and protrusions that clamp the neck portion are provided on at least one of the outer surface of the connecting portion and the inner surface of the cover portion.

3. The infusion device of claim 2, wherein
at least one of the connecting portion and the cover portion is formed such that the recesses and protrusions extend in a circumferential direction of the center axis.

4. The infusion device of claim 1, wherein
the bag is formed by joining edge portions of a pair of synthetic resin sheets that are superimposed on each other at a predetermined width, except for respective portions of the pair of synthetic resin sheets that correspond to an opening of the neck portion.

5. The infusion device of claim 1, wherein
the neck portion is three-dimensionally formed such that an open state is maintained.

6. The infusion device of claim 5, wherein
the connecting portion includes a flange portion that protrudes in the axis orthogonal direction and extends in the circumferential direction of the center axis, and
the flange portion receives an opening end of the neck portion and is fitted to the cover portion.

7. The infusion device of claim 1, wherein
a slope portion inclined downward toward the support portion is provided in a portion of the connecting portion located closer to the support portion.

8. The infusion device of claim 4, wherein
a pair of outer longitudinal grooves that extend in a direction of the center axis are formed so as to face each other on the inner surface of the cover portion that is fitted to the outer surface of the connecting portion, and
a pair of joint allowances that protrude from both sides of the neck portion can be housed in the outer longitudinal grooves when the cover portion is fitted to the connecting portion.

9. The infusion device of claim 1, wherein
a positioning structure that fixes relative positions of the connecting portion and the cover portion in the circumferential direction is provided in each of the connecting portion and the cover portion.

10. The infusion device of claim 1, wherein
an inner longitudinal groove that extends in a direction of the center axis is formed on an outer surface of the connecting portion, and
the inner longitudinal groove constitutes a ventilation passage via which an outer region of the balloon inside the bag communicates with an outside environment.

11. The infusion device of claim 1, wherein
a hole through which a strap can be inserted is formed in the cover portion.

12. The infusion device of claim 1, further comprising:
a tube assembly that transfers the medicinal solution that is pushed out from the balloon,
wherein
the tube assembly includes
a liquid feeding tube one end of which is connected to the liquid outlet and that includes a connector at the other end, and
a flow rate setting portion that sets a flow rate of the medicinal solution that is fed, and
the flow rate setting portion is formed of an orifice tube provided in a portion of the liquid feeding tube and having specific length and flow passage cross section.

13. The infusion device of claim 1, wherein
the bag is formed of a soft material.
